# FASCICULE DE BREVET EUROPEEN

(11) **EP 2 271 605 B1**
(45) Date de publication et mention de la délivrance du brevet: **28.06.2017**
(21) Numéro de dépôt: 09724301.8
(22) Date de dépôt: 27.03.2009
(51) Int. Cl.: B01J 37/26, C07C 17/25, C07C 21/18, B01J 23/86, B01J 37/02, B01J 21/04, B01J 35/10

(54) **PROCEDE DE PREPARATION DE COMPOSES FLUORES**
VERFAHREN ZUR HERSTELLUNG FLUORIERTER VERBINDUNGEN
METHOD FOR PREPARING FLUORINATED COMPOUNDS

(30) Priorité: 28.03.2008 FR 0801729
(43) Date de publication de la demande: 12.01.2011
(73) Titulaire: ARKEMA FRANCE, 92700 Colombes (FR)
(72) Inventeur: DEVIC, Michel, F-69110 Sainte Foy Les Lyon (FR); WENDLINGER, Laurent, F-69510 Soucieu en Jarrest (FR)
(74) Mandataire: Hirsch & Associés
(86) Numéro de dépôt international: PCT/IB2009/005092
(87) Numéro de publication internationale: WO 2009/118630

(56) Documents cités:
- EP-A- 0 644 173
- EP-B- 0 486 333
- EP-B- 0 974 571
- WO-A-2008/030440

## Description

### DOMAINE DE L'INVENTION

L'invention a pour objet un procédé de préparation de composés fluorés, notamment le composé fluoré 1234yf.

### ARRIERE-PLAN TECHNOLOGIQUE

Les hydrofluorocarbones (HFC) et en particulier les hydrofluorooléfines, telles que le 2,3,3,3-tétrafluoro-1-propène (HFO 1234yf) sont des composés connus pour leurs propriétés de réfrigérants et fluides caloporteurs, extinctrices, propulseurs, agents moussants, agents gonflants, diélectriques gazeux, milieu de polymérisation ou monomère, fluides supports, agents pour abrasifs, agents de séchage et fluides pour unité de production d'énergie. A la différence des CFC et des HCFC, qui sont potentiellement dangereux pour la couche d'ozone, les HFOs ne contiennent pas de chlore et donc ne posent pas de problème pour la couche d'ozone.

On connaît plusieurs procédés de fabrication du 1234yf.

WO2008/002499 décrit un procédé de production d'un mélange de 2,3,3,3-tétrafluoro-1-propène (HFO 1234yf) et de 1,3,3,3-tétrafluoro-1-propène (HFO 1234ze) par pyrolyse de 1,1,1,2,3-pentafluoropropane (HFC 245eb). Les températures utilisées sont décrites comme étant de 450°C à 900°C, et en général de 600°C à 750°C qui sont les températures utilisées dans les exemples. Pour une conversion de 245eb supérieure à 50%, le ratio des produits 1234yf :1234ze est de 1,3-1,4 environ.

WO 2008/002500 décrit un procédé de production d'un mélange de 2,3,3,3-tétrafluoro-1-propène (HFO 1234yf) et de 1,3,3,3-tétrafluoro-1-propène (HFO 1234ze) par conversion catalytique de 1,1,1,2,3-pentafluoropropane (HFC 245eb) sur un catalyseur de déhydrofluoration. Le catalyseur de déhydrofluoration préféré est de l'alumine fluorée ou du fluorure d'aluminium (oxyfluorure et fluorure d'aluminium), le catalyseur utilisé dans l'exemple étant de l'oxyfluorure d'aluminium. Sont aussi décrits des catalyseurs de déhydrofluoration choisis parmi les oxydes, fluorures et oxyfluorures de différents métaux, parmi lesquels du chrome et du nickel. Aucun exemple de ces catalyseurs n'est donné. Les températures utilisées sont décrites comme étant de 200°C à 500°C, et en général de 300°C à 450°C, les conversions les plus élevées étant obtenues à 400°C et plus dans les exemples. Pour une conversion de 245eb supérieure à 50%, le ratio des produits 1234yf :1234ze est de 1,9-2,0 environ.

Ces deux demandes précitées visent donc la production d'un mélange contenant une partie substantielle de produit 1234ze.

WO 2007/056194 décrit la préparation de HFO par conversion d'un composé de formule (I) CF₃CHXCH₂X en un composé de formule (II) CF₃CZCHZ, formules dans lesquelles X est indépendamment Cl ou F, et Z est indépendamment H ou F. Il est indiqué que la sélectivité en certains composés, comme notamment le tétrafluoropropène, et plus préférentiellement encore le HFO-1234yf et/ou HFO-1234ze, et d'au moins 70%, de préférence au moins 80%. La réaction a lieu en phase gaz, sur un catalyseur. Les catalyseurs décrits dans cette demande sont des catalyseurs par exemple FeCl₃, oxyfluorure de chrome, Ni (incluant les treillis de Ni mesh), NiCl₂, CrF₃, et leurs mélanges. D'autres catalyseurs décrits sont les catalyseurs supportés sur carbone, les catalyseurs à base d'antimoine, les catalyseurs à base d'aluminium (tel que AlF₃ et Al₂O₃), de palladium, de platine, de rhodium et de ruthénium. Un mode de réalisation préféré est la préparation de 1234yf par déhydrohalogénation de 245eb, notamment sur catalyseur à base de nickel, de carbone ou une combinaison de ceux-ci. La température indiquée comme étant appropriée est comprise entre 450°C et 600°C. Il est indiqué encore une conversion d'au moins 50% et une sélectivité en 1234yf d'au moins 70%, de préférence au moins 80% et plus préférentiellement d'au moins 90%. Les exemples 1-16 de WO2007/056194 donnent plusieurs catalyseurs et conditions de température pour la réaction, avec des conversions et des sélectivités variables. A catalyseur égal, l'augmentation de la température conduit à une augmentation de la conversion mais accompagnée d'une baisse concomitante de la sélectivité, affectant ainsi le rendement en 1234yf.

WO2008/030440 décrit un procédé comprenant une première étape d'hydrogénation de 1,2,3,3,3-pentafluoro-1-propène (1225ye) en 1,1,1,2,3-pentafluoropropane (245eb), qui est ensuite déhydrohalogéné pour conduire au produit recherché le 2,3,3,3-tétrafluoro-1-propène (1234yf). Le catalyseur utilisé dans la réaction de déhydrohalogénation est choisi dans le groupe consistant en fluorure d'aluminium; alumine gamma; alumine fluorée; métal sur fluorure d'alumine; métal sur alumine fluorée; oxydes, fluorures et oxyfluorures de magnésium, zinc et mélanges de magnésium et zinc et/ou aluminium; oxyde de lanthane et oxyde de lanthane fluoré; oxydes de chrome, oxyfluorures de chrome, et trifluorure de chrome cubique; carbone, carbone lavé à l'acide, carbone activé, matériaux carbonés tri-dimensionnels; et composé métallique supporté sur carbone; le métal étant sous forme d'oxyde, de fluorure ou d'oxyfluorure d'au moins un métal du groupe consistant en sodium, potassium, rubidium, césium, yttrium, lanthane, cérium, praséodyme, néodyme, samarium, chrome, fer, cobalt, rhodium, nickel, cuivre, zinc, et leurs mélanges. Les exemples mentionnent comme métal un mélange Cr/Co. La conversion obtenue est d'environ 50%, pour une température de 250°C et un temps de contact de 30 secondes.

Le document WO2007/056128 décrit la réaction de déhydrohalogénation de composés de formule (I) CF₃CFₙCHₘXₐ₋ₘ en un composé de formule (II) CF₃CZ=CHZ. Cette réaction est notamment conduite en présence d'un agent réducteur, l'hydrogène.

De même, on connaît les réactions de déhydrofluoration de 1,1,2,2,3,3-hexafluoropropane (HFC 236ea) en 1,2,3,3,3-pentafluoropropène-1 (1225ye).

Le document US-P-5396000 décrit la préparation de 1,1,1,2,3-pentafluoropropane par déhydrohalogénation catalytique de 1,1,1,2,3,3-hexafluoropropane (236ea) en 1,2,3,3,3-pentafluoropropène-1 (1225ye), suivi d'une hydrogénation pour produire le composé recherché. La déshydrohalogénation du 236ea en 1225ye est effectuée en phase gazeuse, sur un catalyseur à base d'alumine fluorée. La sélectivité en 1225ye n'est pas indiquée.

Le document US-P-5679875 décrit la préparation de 1,1,1,2,3-pentafluoropropane par déhydrohalogénation catalytique de 1,1,1,2,3,3-hexafluoropropane (236ea) en 1,2,3,3,3-pentafluoropropène-1 (1225ye), suivi d'une hydrogénation pour produire le composé recherché. Les réactions sont effectuées en phase gaz. Le catalyseur de déhydrohalogénation est l'oxyde ou oxyfluorure de chrome III, ou du carbone. De l'oxygène peut être ajouté au courant de 1,1,1,2,3,3-hexafluoropropane pour maintenir l'activité catalytique au cours du temps. Les sélectivités sont élevées, mais la conversion n'est pas toujours très élevée.

EP-A-974571 décrit un procédé de déhydrofluoration du 1,1,1,3,3-pentafluoropropane en 1,1,1,3-tétrafluoro-2-propène.

Il existe donc un besoin d'un procédé de préparation de 1234yf et de 1225ye à partir de 245eb et de 236ea, respectivement, et ce avec une sélectivité élevée pour une conversion élevée, et par là un rendement élevé. Il existe aussi un besoin d'un procédé catalytique présentant une productivité élevée.

### RESUME DE L'INVENTION

L'invention fournit donc un procédé de déhydrofluoration sélective sur un catalyseur mixte à base de chrome et de nickel sur un support à base d'aluminium.

L'invention fournit encore un procédé de déhydrofluoration d'un composé de formule (I) CF₃-CHF-CHFX, dans lequel X est hydrogène en un composé de formule (II) CF₃-CF=CHX, sur un catalyseur mixte à base de chrome et de nickel sur un support à base d'aluminium.

Selon des modes de réalisation :
- le composé de formule (I) est le composé de formule (Ib) CF₃-CHF-CH₂F et le composé de formule (II) est le composé de formule (IIb) CF₃-CF=CH₂.
- le catalyseur mixte comprend un oxyde, un halogénure ou un oxyhalogénure de chrome en mélange avec un oxyde, un halogénure ou un oxyhalogénure de nickel.
- le catalyseur mixte comprend un fluorure ou un oxyfluorure de chrome en mélange avec un fluorure ou un oxyfluorure de nickel.
- le support à base d'aluminium est un halogénure ou un oxyhalogénure d'aluminium.
- le support à base d'aluminium est un fluorure ou un oxyfluorure d'aluminium.
- le catalyseur comprend en poids de 0,5 à 20% de chrome et de 0,5 à 20% de nickel, ces éléments étant présents selon un ratio molaire entre 0,5 et 5.
- le procédé est mis en oeuvre en phase gazeuse.
- le procédé est mis en oeuvre à une température comprise entre 150°C et 600°C, de préférence entre 300 et 500°C et avantageusement entre 300 et 400°C.
- le procédé est mis en oeuvre avec un temps de contact compris entre 0,1 et 100 secondes, de préférence entre 1 et 50 secondes et avantageusement entre 2 et 20 secondes.
- le procédé est mis en oeuvre en présence d'hydrogène, le ratio molaire H₂/1,1,1,2,3-pentafluoropropane étant compris entre 0,3 et 30, notamment entre 0,5 et 20, avantageusement entre 1 et 10.

L'invention fournit encore une utilisation d'un catalyseur mixte à base de chrome et de nickel sur un support à base d'aluminium comme catalyseur de réaction de déhydrofluoration sélective.

### DESCRIPTION DETAILLEE DE MODES DE REALISATION

L'invention utilise un catalyseur mixte de chrome et de nickel.

Ce catalyseur mixte, contient à la fois du chrome et du nickel. Le ratio molaire Cr :Ni, par rapport à l'élément métallique, est généralement compris entre 0,5 et 5, par exemple entre 0,7 et 2, notamment voisin de 1. Le catalyseur peut contenir en poids de 0,5 à 20% de chrome et de 0,5 à 20% de nickel et, de préférence, entre 2 et 10% de chacun des métaux.

Le métal peut être présent sous forme métallique ou sous forme de dérivés, notamment oxyde, halogénure ou oxyhalogénure, ces dérivés, notamment halogénure et oxyhalogénure, étant obtenus par activation du métal catalytique. Bien que l'activation du métal ne soit pas nécessaire, elle est préférée.

Le support est à base d'aluminium. On peut citer plusieurs supports possibles comme l'alumine, l'alumine activée ou les dérivés d'aluminium. Ces dérivés d'aluminium sont notamment des halogénures ou oxyhalogénures d'aluminium, décrits par exemple dans US-P-4902838, ou obtenus par le procédé d'activation décrit ci-dessous.

Le catalyseur peut comprendre le chrome et du nickel sous une forme non-activée ou sous forme activée, sur un support qui a subi aussi l'activation du métal ou non.

Le catalyseur peut être préparé à partir d'alumine (en général une alumine dite activée; cette alumine activée est une alumine à porosité élevée, et est distincte de l'alumine ayant subi le traitement d'activation du métal). Dans une première étape l'alumine est transformée en fluorure d'aluminium ou en mélange de fluorure d'aluminium et d'alumine, par fluoration à l'aide d'air et d'acide fluorhydrique, le taux de transformation de l'alumine en fluorure d'aluminium dépendant essentiellement de la température à laquelle est effectuée la fluoration de l'alumine (en général entre 200°C et 450°C, de préférence entre 250°C et 400°C). Le support est ensuite imprégné à l'aide de solutions aqueuses de sels de chrome et de nickel ou à l'aide de solutions aqueuses d'acide chromique, de sel de nickel et de méthanol (servant de réducteur au chrome). Comme sels de chrome et de nickel, on peut employer des chlorures, ou d'autres sels tels que, par exemple, les oxalates, formiates, acétates, nitrates et sulfates ou le bichromate de nickel, pour autant que ces sels soient solubles dans la quantité d'eau susceptible d'être absorbée par le support. Le catalyseur peut aussi être préparé par imprégnation directe de l'alumine (qui en général est activée) à l'aide des solutions des composés de chrome et de nickel ci-dessus mentionnées. Dans ce cas, la transformation d'au moins une partie (par exemple 70% ou plus) de l'alumine en fluorure d'aluminium ou oxyfluorure d'aluminium s'effectue lors de l'étape d'activation du métal du catalyseur.

Les alumines activées susceptibles d'être utilisées pour la préparation du catalyseur sont des produits bien connus, disponibles dans le commerce. Elles sont généralement préparées par calcination d'hydrates d'alumine (hydroxydes d'aluminium) à une température comprise entre 300°C et 800°C. Les alumines (activées ou non) peuvent contenir des teneurs importantes (jusqu'à 1000 ppm) de sodium sans que cela nuise aux performances catalytiques.

De préférence, mais sans que cela soit nécessaire, le catalyseur est conditionné ou activé, c'est-à-dire transformé en constituants actifs et stables (aux conditions réactionnelles) par une opération préalable dite d'activation. Ce traitement peut être réalisé soit "in situ" (dans le réacteur de déhydrofluoration) ou bien dans un appareillage adéquat conçu pour résister aux conditions d'activation.

Cette étape d'activation comprend en général les étapes suivantes :
- Une étape de séchage. Cette étape de séchage est effectuée à haute température (250°C à 450°C, de préférence 300°C à 350°C) en général sous courant d'azote ou d'air. Cette étape peut être éventuellement précédée dans un premier temps d'une première étape de séchage à basse température (100°C à 150°C, de préférence 110°C à 120°C) en présence d'air ou d'azote. La durée de l'étape de séchage peut être comprise entre 1 et 50 heures.
- Une étape de fluoration. Cette étape de fluoration est mise en oeuvre à basse température (180°C à 350°C) au moyen d'un mélange d'acide fluorhydrique et d'azote, en contrôlant la teneur en HF de façon que la température ne dépasse pas 350°C. La durée de l'étape de fluoration peut être comprise entre 1 et 50 heures.
- Eventuellement une étape de finition sous courant d'acide fluorhydrique pur ou dilué par de l'azote à une température pouvant aller jusqu'à 450°C. La durée de l'étape de finition peut être comprise entre 1 et 15 heures.

Pendant cette opération, les précurseurs catalytiques (par exemple halogénures de nickel et de chrome, chromate ou bichromate de nickel, oxyde de chrome) sont transformés en fluorures et/ou oxyfluorures correspondants, ce qui entraîne un dégagement d'eau et/ou d'acide chlorhydrique. L'analyse chimique des éléments (chrome, nickel, fluor, aluminium, oxygène), après cette activation, permet de vérifier la composition minérale du catalyseur.

Un tel catalyseur est décrit dans EP-A-486333, en particulier page 3, lignes 11-48, exemples 1A, 2A et 4A, passages auxquels il est renvoyé. Ce catalyseur est utilisé dans ce document comme un catalyseur de fluoration, dans une réaction de fluoration catalytique du chloro-1-trifluoro-2,2,2-éthane (133a) avec du HF gazeux pour conduire au tétrafluoro-1,1,1,2-éthane (134a).

La réaction de déhydrofluoration est la réaction dans laquelle HF est éliminé d'un composé de départ, conduisant à la création d'une double liaison dans le composé final. La réaction considérée est sélective, en ce sens que le composé de départ (typiquement un composé hydrofluorocarboné ayant jusque 4 atomes de carbone) présente une stéréochimie telle que l'élimination de HF conduise à au moins deux isomères de position. Les composés fluorocarbonés de départ sont en particulier ceux comprenant un groupe terminal trifluorométhyle.

La réaction de déhydrofluoration est la réaction de déhydrofluoration d'un composé de formule (I) CF₃-CHF-CHFX, dans lequel X est hydrogène en un composé de formule (II) CF₃-CF=CHX.

Selon un premier mode de réalisation, le composé de formule (I) est le composé de formule (Ia) CF₃-CHF-CHF₂ (236ea) et le composé de formule (II) est le composé de formule (IIa) CF₃-CF=CHF (1225ye).

Selon un second mode de réalisation, le composé de formule (I) est le composé de formule (Ib) CF₃-CHF-CH₂F (245eb) et le composé de formule (II) est le composé de formule (IIb) CF₃-CF=CH₂ (1234yf).

La réaction de déhydrofluoration est mise en oeuvre en général en phase gazeuse.

Le catalyseur peut être présent sous toute forme appropriée, par exemple sous forme de lit fixe ou fluidisé, de préférence en lit fixe. La direction de flux peut être de haut en bas ou de bas en haut.

La température peut être comprise entre 150°C et 600°C, de préférence entre 300 et 500°C et avantageusement entre 300 et 400°C.

La pression peut être atmosphérique, ou inférieure ou supérieure à cette pression atmosphérique.

Le temps de contact (rapport entre le volume de catalyseur et le flux total de la charge) est en général compris entre 0,1 et 100 secondes, de préférence entre 1 et 50 secondes, en particulier pour le 236ea et avantageusement entre 2 et 20 secondes, en particulier pour le 245eb.

Un gaz diluant (azote, hélium ou argon) peut être utilisé dans la réaction. De l'hydrogène peut aussi être injecté, par exemple en continu, ou en discontinu. Le ratio molaire H₂/245eb peut varier dans de grandes mesures, notamment entre 0,3 et 30, notamment entre 0,5 et 20, avantageusement entre 1 et 10.

La réaction est mise en oeuvre dans un réacteur dédié aux réactions impliquant des halogènes. De tels réacteurs sont connus de l'homme du métier, et peuvent comprendre des revêtements intérieurs à base par exemple de Hastelloy®, Inconel®, monel® ou de fluoropolymères. Le réacteur peut aussi comprendre des moyens d'échange de chaleur, si besoin.

L'alimentation en réactifs peut se fait en général en continu, ou peut être étagée le cas échéant.

Le produit de la réaction, contenant notamment le 1234yf HF et des sous-produits éventuels et des produits de départ n'ayant pas réagi, est séparé de façon classique. Les éventuels réactifs n'ayant pas réagi sont avantageusement recyclés dans le procédé.

Dans le procédé, la conversion est de plus de 50%, de préférence plus de 70% et avantageusement plus de 80%. La sélectivité est très élevée, en général de plus de 80%, de préférence plus de 90% et avantageusement plus de 95%. Le rendement est en général supérieur à 80%.

On rappellera que :
- le taux de conversion est le% du produit de départ ayant réagi (nombre mole de produit départ ayant réagi/nombre mole produit départ introduit);
- la sélectivité en produit recherché est le ratio nombre de mole de produit recherché formé/nombre mole produit départ ayant réagi;
- le rendement en produit recherché est le ratio nombre de mole de produit recherché formé/nombre mole produit départ introduit, le rendement en produit recherché pouvant encore être défini comme le produit de la conversion et de la sélectivité.
- le temps de contact est l'inverse de la vitesse spatiale VVH (ou GHSV en langue anglaise)
- la vitesse spatiale est le rapport entre le débit volumique total sur le volume du lit catalytique, dans
les conditions normales de température et de pression.

### EXEMPLES

Les exemples suivants illustrent l'invention sans la limiter.

### Exemple 1. Préparation du catalyseur.

Le catalyseur utilisé est un catalyseur Ni-Cr/AlF₃ préparé comme suit.

Dans un évaporateur rotatif, on place 343 g d'un support obtenu dans une étape précédente par fluoration d'alumine GRACE HSA en lit fixe vers 280°C à l'aide d'air et d'acide fluorhydrique (concentration volumique de 5 à 10% de cet acide dans l'air). L'alumine GRACE HSA de départ présente les caractéristiques physicochimiques suivantes :
- forme : billes de 0,5-2 mm de diamètre
- surface BET : 220 m²/g
- volume poreux : 1,3 cm³/g

On prépare par ailleurs deux solutions aqueuses séparées :
(a) solution chromique additionnée de chlorure de nickel contenant :

| | |
|---|---|
| - anhydride chromique | 55 g |
| - chlorure de nickel hexahydraté | 130 g |
| - eau | 63 g |

(b) Solution méthanolique contenant :

| | |
|---|---|
| - méthanol | 81 g |
| - eau | 8 g |

Ces deux solutions sont introduites simultanément à une température de 40°C sous pression atmosphérique et en 2 heures environ, sur le support en agitation. Après une étape de maturation sous azote, le catalyseur est séché sous azote, puis sous vide à 65°C puis vers 90°C pendant 6 heures.

On charge 500 g de solide imprégné dans un réacteur tubulaire en inconel. Le catalyseur est tout d'abord séché sous balayage d'azote à basse température puis jusqu'à 320°C, à pression atmosphérique. Il est ensuite fluoré en présence d'un mélange acide fluorhydrique / azote (concentration volumique de 5 à 10% de cet acide dans l'azote) à 320°C puis jusqu'à 390°C. L'alimentation d'HF est alors coupée. Le balayage à l'azote est maintenu pendant 15 minutes à 390°C puis le catalyseur est refroidi jusqu'à 60°C sous balayage d'azote.

Les caractéristiques du catalyseur après activation sont les suivantes :
- surface BET : 40 m²/g
- volume poreux : 0,4 cm³/g
- composition chimique pondérale :
   ▪ Al : 25%
   ▪ F : 58%
   ▪ Cr : 5,3%
   ▪ Ni : 6,4%

### Exemple 2. Déhydrofluoration de 245eb.

On utilise un réacteur d'un volume de 50 cm³, de diamètre intérieur 2,1 cm, contenant 20 g de catalyseur de l'exemple 1 sous forme d'un lit fixe d'une hauteur de 6,5 cm. La pression est de une atmosphère. Le réactif est mélangé avec de l'hydrogène et injecté dans le réacteur, préalablement chauffé à la température de réaction. Un échantillon de l'effluent gazeux est ensuite analysé par chromatographie phase gazeuse.

On obtient alors les résultats suivants (RM signifie ratio molaire).

| Temp. | Temps contact | RM H₂/245eb | Conversion | Sélectivité 1234yf | Sélectivité 1234ze | Productivité 1234yf kg/h/m³ cata |
|---|---|---|---|---|---|---|
| 377°C | 8,8 sec | 1,4 | 99,8% | 89,6% | 9,1% | 773 |

On note que la productivité avec le catalyseur mixte est très élevée, tout en assurant une conversion et une sélectivité aussi très élevée.

### Exemple 3. Déhydrofluoration de 245eb en 1234yf.

On utilise un réacteur d'un volume de 25 cm³, contenant 10 g de catalyseur de l'exemple 1 sous forme d'un lit fixe. La pression est de une atmosphère.

On obtient alors les résultats suivants (RM signifie ratio molaire).

| Temp. | Temps contact | RM H₂/245eb | Conversion | Sélectivité 1234yf | Sélectivité 1234ze | Productivité 1234yf kg/h/m³ cata |
|---|---|---|---|---|---|---|
| 373°C | 8,9 sec | 3 | 87% | 90% | 9% | 436 |

On note encore que la productivité avec le catalyseur mixte est très élevée, tout en assurant une conversion et une sélectivité aussi très élevée.

## Revendications

1. Procédé de déhydrofluoration sélective d'un composé de formule (Ib) CF₃-CHF-CH₂F, en un composé de formule (IIb) CF₃-CF=CH₂, sur un catalyseur mixte à base de chrome et de nickel sur un support à base d'aluminium.

2. Procédé selon la revendication 1, dans lequel le catalyseur mixte comprend un oxyde, un halogénure ou un oxyhalogénure de chrome en mélange avec un oxyde, un halogénure ou un oxyhalogénure de nickel.

3. Procédé selon l'une des revendications 1 ou 2, dans lequel le catalyseur mixte comprend un fluorure ou un oxyfluorure de chrome en mélange avec un fluorure ou un oxyfluorure de nickel.

4. Procédé selon l'une des revendications 1 à 3, dans lequel le support à base d'aluminium est un halogénure ou un oxyhalogénure d'aluminium.

5. Procédé selon l'une des revendications 1 à 4, dans lequel le support à base d'aluminium est un fluorure ou un oxyfluorure d'aluminium.

6. Procédé selon l'une des revendications 1 à 5, dans lequel le catalyseur comprend en poids de 0,5 à 20% de chrome et de 0,5 à 20% de nickel, ces éléments étant présents selon un ratio molaire entre 0,5 et 5.

7. Procédé selon l'une des revendications 1 à 6, mis en oeuvre en phase gazeuse.

8. Procédé selon l'une des revendications 1 à 7, mis en oeuvre à une température comprise entre 150°C et 600°C, de préférence entre 300 et 500°C et avantageusement entre 300 et 400°C.

9. Procédé selon l'une des revendications 1 à 8, mis en oeuvre avec un temps de contact compris entre 0,1 et 100 secondes, de préférence entre 1 et 50 secondes et avantageusement entre 2 et 20 secondes.

10. Procédé selon l'une des revendications 1 à 9, mis en oeuvre en présence d'hydrogène, le ratio molaire H₂/1,1,1,2,3-pentafluoropropane étant compris entre 0,3 et 30, notamment entre 0,5 et 20, avantageusement entre 1 et 10.

11. Utilisation d'un catalyseur mixte à base de chrome et de nickel sur un support à base d'aluminium comme catalyseur de réaction de déhydrofluoration sélective d'un composé de formule (Ib) CF₃-CHF-CH₂F, en un composé de formule (IIb) CF₃-CF=CH₂.

## Patentansprüche

1. Verfahren zur selektiven Dehydrofluorierung einer Verbindung der Formel (Ib) CF₃-CHF-CH₂F in eine Verbindung der Formel (IIb), CF₃-CF=CH₂ auf einem Mischkatalysator auf Chrom- und Nickelbasis auf einem Träger auf Aluminiumbasis.

2. Verfahren nach Anspruch 1, wobei der Mischkatalysator ein Chromoxid, ein Chromhalogenid oder ein Chromoxihalogenid umfasst, im Gemisch mit einem Nickeloxid, einem Nickelhalogenid oder einem Nickeloxihalogenid.

3. Verfahren nach einem der Ansprüche 1 oder 2, wobei der Mischkatalysator ein Chromfluorid oder ein Chromoxifluorid umfasst, im Gemisch mit einem Nickelfluorid oder einem Nickeloxifluorid.

4. Verfahren nach einem der Ansprüche 1 bis 3, wobei der Träger auf Aluminiumbasis ein Aluminiumhalogenid oder Aluminiumoxihalogenid darstellt.

5. Verfahren nach einem der Ansprüche 1 bis 4, wobei der Träger auf Aluminiumbasis ein Aluminiumfluorid oder Aluminiumoxifluorid darstellt.

6. Verfahren nach einem der Ansprüche 1 bis 5, wobei der Katalysator 0,5-20% an Gew.-% Chrom und 0,5 bis 20% an Gew.-% Nickel umfasst, wobei diese Elemente in einem Molverhältnis zwischen 0,5 und 5 vorhanden sind.

7. Verfahren nach einem der Ansprüche 1 bis 6, das in der Gasphase durchgeführt wird.

8. Verfahren nach einem der Ansprüche 1 bis 7, das durchgeführt wird bei einer Temperatur zwischen 150 °C und 600 °C, vorzugsweise zwischen 300 und 500 °C und vorteilhafterweise zwischen 300 und 400 °C.

9. Verfahren nach einem der Ansprüche 1 bis 8, das durchgeführt wird mit einer Kontaktzeit zwischen 0,1 und 100 Sekunden, vorzugsweise zwischen 1 und 50 Sekunden und vorteilhafterweise zwischen 2 und 20 Sekunden.

10. Verfahren nach einem der Ansprüche 1 bis 9, das durchgeführt wird in Gegenwart von Wasserstoff, wobei das Molverhältnis H₂/1,1,1,2,3-Pentafluorpropan zwischen 0,3 und 30 liegt, insbesondere zwischen 0,5 und 20, vorteilhafterweise zwischen 1 und 10.

11. Verwendung eines Mischkatalysators auf Chrom- und Nickelbasis auf einem Träger auf Aluminiumbasis als Reaktionskatalysator der selektiven Dehydrofluorierung einer Verbindung der Formel (Ib) CF₃-CHF-CH₂F in eine Verbindung der Formel (IIb) CF₃-CF=CH₂.

## Claims

1. Selective dehydrofluorination process of a compound of formula (Ib) CF₃-CHF-CH₂F to a compound of formula (IIb) CF₃-CF=CH₂, on a mixed catalyst based on chromium and nickel on a support based on aluminum.

2. Process according to claim 1, in which the mixed catalyst comprises a chromium oxide, halide or oxyhalide in a mixture with a nickel oxide, halide or oxyhalide.

3. Process according to claims 1 or 2, in which the mixed catalyst comprises a chromium fluoride or oxyfluoride in a mixture with a nickel fluoride or oxyfluoride.

4. Process according to claims 1 to 3, in which the support based on aluminum is an aluminum halide or oxyhalide.

5. Process according to claims 1 to 4, in which the support based on aluminum is an aluminum fluoride or oxyfluoride.

6. Process according to claims 1 to 5, in which the catalyst comprises by weight 0.5 a 20% chromium and 0.5 to 20% nickel, these elements being present according to a molar ratio between 0.5 and 5.

7. Process according to claims 1 to 6, implemented in gas phase.

8. Process according to one of claims 1 to 7, implemented at a temperature comprised between 150°C and 600°C, preferably between 300 and 500°C and advantageously between 300 and 400°C.

9. Process according to one of claims 1 to 8, implemented with a contact time comprised between 0.1 and 100 seconds, preferably between 1 and 50 seconds and advantageously between 2 and 20 seconds.

10. Process according to one of claims 1 to 9, implemented in the presence of hydrogen, the molar ratio H₂/1,1,1,2,3-pentafluoropropane being comprised between 0.3 and 30, in particular between 0.5 and 20, advantageously between 1 and 10.

11. Use of a mixed catalyst based on chromium and nickel on a support based on aluminum as a catalyst for a selective dehydrofluorination reaction of a compound of formula (Ib) CF₃-CHF-CH₂F, to a compound of formula (IIb) CF₃-CF=CH₂.
